# EUROPÄISCHE PATENTANMELDUNG

(11) **EP 1 772 139 A1**
(43) Veröffentlichungstag der Anmeldung: **11.04.2007**
(21) Anmeldenummer: 06121941.6
(22) Anmeldetag: 09.10.2006
(51) Int. Cl.: A61K 8/97, A61Q 19/00

(54) **Wasserhaltige kosmetische Zubereitung mit gelösten Flavonolignanen**

(30) Priorität: 10.10.2005 DE 102005048780
(71) Anmelder: Beiersdorf AG, 20253 Hamburg (DE)
(72) Erfinder: Eckert, Julia, 20257, Hamburg (DE); Kolbe, Ludger, 21255, Dohren (DE); Kruse, Inge, 20146, Hamburg (DE); Raschke, Thomas, 25421, Pinneberg (DE)

(57) **Zusammenfassung**

Die vorliegende Erfindung betrifft wasserhaltige kosmetische Zubereitungen mit gelösten Flavonolignanen, insbesondere Emulsionen mit Silymarin.

## Beschreibung

Die vorliegende Erfindung betrifft wasserhaltige kosmetische Zubereitungen mit gelösten Flavonolignanen, insbesondere Emulsionen mit Silymarin.

Es ist allgemein bekannt, dass schwerlösliche Wirkstoffe aus der Gruppe der Flavonolignane wie die Früchte der Mariendistel (Silybum marianum) ein komplexes Gemisch von Flavonolignanen enthalten, das als Silymarin bezeichnet wird. Die Leitstruktur, der eine radikalfangende und antioxidative Wirkung zugeschrieben wird, ist das Silibinin (siehe Max Wichtel, Teedrogen und Phytopharmaka, wissenschaftliche Verlagsgesellschaft mbH Stuttgart, 4. Auflage S. 107-111; Hagers Handbuch der Pharmazeutischen Praxis, 5. Auflage, Springer Verlag, Stoffe P-Z, S. 615-617).

Aufgrund seiner Zellschutzwirkung sind schwerlösliche Wirkstoffe aus der Gruppe der Flavonolignane wie Mariendistelextrakt und die Reinsubstanz Silibinin für den Einsatz in Kosmetika als Wirkstoffe interessant (siehe EP 236218 B1).

Kosmetische Zubereitungen enthalten oft Öl und Wasser. Sie liegen beispielsweise als Emulsionen oder ölhaltige Gele vor.

Der Einsatz von Flavonolignanen in solchen kosmetischen Zubereitungen ist jedoch durch die geringe Löslichkeit dieser Substanzen limitiert.

Silymarin und andere Flavonolignane sind so gut wie unlöslich in Wasser und auch in fast allen gängigen Lipiden nicht löslich. Allein in Polyolen kann beispielsweise der Wirkstoff Silymarin gelöst werden. Reine Polyole und Ethoxydiglycol sind in der Lage, Silymarin, Silyphos und Silibinin zu lösen. Dieser Effekt wird in Emulsionen jedoch verwässert und die Kristallisationsneigung nimmt wieder zu. Deshalb war es bisher nicht einmal möglich, 0,1 Gew.% Silymarin in kosmetischen Formulierungen einzuarbeiten, ohne dass der Wirkstoff in der Endformulierung auskristallisierte.

Eine Möglichkeit, die Wasserlöslichkeit von schwerlöslichen Wirkstoffen aus der Gruppe der Flavonolignane wie Silymarin bzw. Silibinin zu verbessern, ist die Komplexierung mit Cyclodextrinen. Solche Einschlussverbindungen werden in EP 0422497 B1 beansprucht und in folgender Literaturstelle beschrieben: BollChim Farm. 1992 May; 131 (5): 205-9. Der Einsatz von Cyclodextrinen weist aber folgendes Problem auf: Die Komplexierung ist nur möglich, wenn der pH Wert zumindest kurzfristig auf > 7 eingestellt wird. Im Alkalischen kann sind die Strukturen jedoch nicht stabil. Darüber hinaus ist das Verfahren sehr aufwendig. Bisher wird noch kein CD komplexiertes Material angeboten.

### Erfindung

Es wurden nun gefunden, dass die Nachteile des Standes der Technik für den Fachmann nicht vorhersehbar überwunden werden durch wasserhaltige kosmetische Zubereitung enthaltend mindestens einen schwerlöslichen Wirkstoff aus der Gruppe der Flavonolignane, mindestens ein bei Raumtemperatur flüssiges, synthetisches Ester-Öl mit besonders hohem Gehalt an Esterbindungen und einen hydrophilen Emulgator aus der Gruppe Zuckerderivate, PEG-Ester und / oder Lecithine.

Diese zeichnen sich aufgrund der bioverfügbaren Flavonolignane durch eine verbesserte Hautpflegeleistung aus.

Es wurde weiter gefunden, dass es bevorzugt ist, wenn der oder die schwerlöslichen Wirkstoffe aus der Gruppe der Flavonolignane Silymarin, Silibinin, Silychristin, Isosilybin, Silydianin, besonders bevorzugt Silymarin und/oder Silibinin gewählt werden.

Weiterhin ist es bevorzugt, wenn der schwerlösliche Wirkstoff aus der Gruppe der Flavonolignane in Konzentrationen von 0,0001 bis 5 Gew.-% bezogen auf das Gewicht der gesamten Zubereitung vorliegen.

Weiterhin ist es bevorzugt, wenn das oder die bei Raumtemperatur flüssigen, synthetischen Ester-Öle mit besonders hohem Gehalt an Esterbindungen aus der Gruppe der Alkylbenzoate, Alkyllaktate, Alkylmalate, Isopropylpalmitat, Propylene Glycol Isostearate, Propylene Glycol Monolaurate, Butylene Glycol Caprylate/ Caprate, Dibutyl Adipate, Propylene Glycol Monolaurate, Pentaerythrityl Tetraisostearate gewählt werden.

Weiterhin ist es bevorzugt, wenn das oder die bei Raumtemperatur flüssigen, synthetischen Ester-Öle mit besonders hohem Gehalt an Esterbindungen in Konzentrationen von 1 bis 30 Gew.-% bezogen auf das Gewicht der gesamten Zubereitung vorliegen.

Weiterhin ist es bevorzugt, wenn der hydrophile Emulgator aus der Gruppe Cetearylglucoside, Polyglycerol-3 Methyl Glucose Distearate, PEG- Ester wie PEG-40-Stearate und/oder Lecithine gewählt wird.

Weiterhin ist es bevorzugt, wenn die Zubereitung mindestens 5 Gew.-% Öl enthält, bezogen auf das Gewicht der gesamten Zubereitung.

Weiterhin ist es bevorzugt, wenn die Zubereitung eine Emulsion ist.

Weiterhin ist es bevorzugt, wenn ein sprühgetrockneter Extrakt eingesetzt wird.

Die Erfindung umfasst auch eine erfindungsgemäße Zubereitung zur oralen Applikation und die Verwendung von bei Raumtemperatur flüssigen synthetischen Ester-Ölen mit besonders hohem Gehalt an Esterbindungen und hydrophilen Emulgatoren aus der Gruppe Zuckerderivate oder Lecithine zur Verbesserung der Löslichkeit und/oder Verringerung der Kristallisationsneigung von in wasserhaltigen Zubereitungen schwerlöslichen Wirkstoffe aus der Gruppe der Flavonolignanen, bevorzugt Silymarin, Silibinin, Silychristin, Isosilybin, Silydianin, besonders bevorzugt Silymarin und/oder Silibinin in wasserhaltigen Zubereitungen.

Das Weglassen eines einzelnen Bestandteils beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Bei den wasserhaltigen kosmetischen Zubereitungen enthaltend mindestens einen schwerlöslichen Wirkstoff aus der Gruppe der Flavonolignane, mindestens ein bei Raumtemperatur flüssiges, synthetisches Ester-Öl mit besonders hohem Gehalt an Esterbindungen und einen hydrophilen Emulgator aus der Gruppe der Zuckerderivate, Lecithine oder PEG-Ester kann es sich um O/W Emulsionen und W/O Emulsionen handeln. Weitere mögliche Anwendungsformen sind Sprays, Sticks, Pflaster, Gele, Kapseln, Food Supplements, Getränkekonzentrate, Tonics, Lotions, Tücher und Badekonzentrate.

Es ist bei all diesem im Einzelfalle möglich, dass die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so dass er weiß, dass bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

Einarbeitung von schwerlöslichen Flavonolignanen aus Silybum Marianum in eine kosmetische Zubereitung (Labormaßstab):

**Beispielrezeptur (After Shave auf Basis einer O/W Emulsion):**

| Fettphase: | Wirkstoffphase: | Wasserphase: |
|---|---|---|
| Cetearylglucosid :1.5% | C12-C13 Alkyl Lactate: 2% | Glycerin: 7.5% |
| Tocopheryl Acetate: 0.5% | C12-C13 Alkyl Benzoate: 2% | Methylparaben: 0.3% |
| Phenoxyethanol 0.4% | Phosphatidylcholin: 0.075% | Panthenol: 2% |
| | Silibinin: 0.025% | Aqua: ad 100% |

| Parfumphase: | Verdickerphase: | |
|---|---|---|
| Parfum: 0.6% | Carbomer: 0.4% | |

### Herstellung der Wirkstoffphase (1 kg Ansatz, 0.025% Silibinin)

1g einer Mischung aus ca. 25% Silibinin und 75% Phosphatidylcholin wird unter Rühren zu 20g C12-15 Alkyl Benzoate und 20g C 12-15 Alkyl Lactate gegeben. Diese Wirkstoffphase wird unter Rühren auf > 70°C erwärmt (max. 90°C) bis eine klare Lösung entsteht (ca. 15 Minuten heiß Rühren).

Die so hergestellte Wirkstoffphase wird in kosmetische Zubereitungen, z.B. wie folgt eingearbeitet:
■ Fettphase (Aluschüssel) und Wasserphase (Erlmeyerkolben) auf ca. 80°C erhitzen,
■ Fettphase vorlegen und Wirkstoffphase (80 °C) zufügen,
■ heiße Wasserphase unter ständigem Rühren sukkzesive zufügen(60 U/min),
■ nach der Phasenvereinigung Natronlauge zugeben (ca. 70°C).
■ Anschließend 1x auf Stufe 1 (2800 U/min, Spaltbreite: 0,2 mm, einfacher Durchlauf) mit Homocenta (Rotor/ Stator Prinzip) homogenisieren,
■ bei Raumtemperatur Emulsion auf ca.30°C kalt rühren, Parfumphase zugeben und 10 Minuten rühren (30-40 U/min),
■ bei 25°C erneut 1 x auf Stufe 1 (2800 U/min, Spaltbreite: 0,2 mm, einfacher Durchlauf) mit Homocenta (Rotor/ Stator Prinzip) homogenisieren.

Alternativ zu C 12-15 Alkyl Benzoate und C 12-15 Alkyl Lactate können folgende Öle verwendet werden: Propylene Glycol Isostearate, Alkylmalate, Isopropylpalmitat, Propylene Glycol Monolaurate, Butylene Glycol Caprylate/ Caprate, Dibutyl Adipate, Pentaerythrityl Tetraisostearate.

Als Wirkstoff können neben Silibinin auch Silymarin, Silychristin, Isosilybin, Silydianin oder andere Flavonolignane verwendet werden.

| Beispiel 1 | Gew.-% |
|---|---|
| O/W-Nachtcreme | |
| Cetearylglucosid | 2 |
| Sheabutter | 2 |
| Glycerylstearat | 1 |
| Stearylalkohol | 2 |
| Cetylalkohol | 2 |
| C₁₂₋₁₅ Alkylbenzoat | 2 |
| Caprylsäure/Caprinsäuretriglyceride | 2 |
| Ethylhexylkokosfettsäureester | 2 |
| Cyclometicone | 3 |
| Dicaprylylether | 2 |
| Tocopherylacetat | 1 |
| Ubichinon (Q10) | 0,1 |
| Natriumascorbylphosphat | 0,1 |
| Extrakt aus Silybum Marianum | 0,2 |
| p-Hydroxybenzoesäurealkylester (Paraben) | 0,6 |
| Ethylhexylglycerin | 0,5 |
| Aristoflex AVC (Ammonium Polyacryldimethyltauramid/Vinylformamid-copolymer) | 0,3 |
| EDTA | 0,2 |
| Glycerin | 10 |
| wasser- und/oder öllösliche Farbstoffe | 0,05 |
| Füllstoffe/ Additive (SiO₂, BHT) | 0,2 |
| Nylon | 2 |
| Folsäure | 0.01 |
| Parfüm | q.s. |
| Wasser | ad 100 |

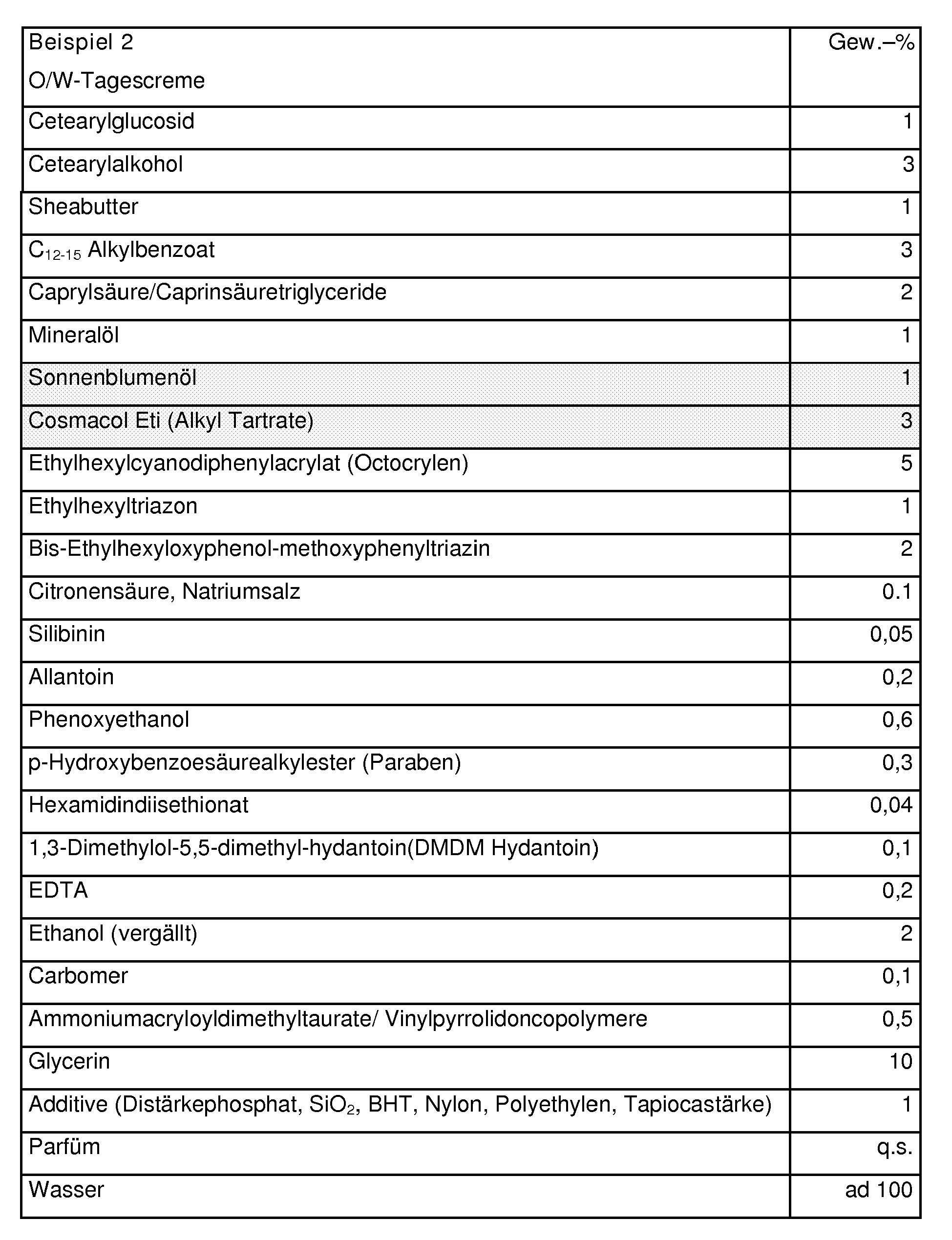

| Beispiel 3 | Gew.-% |
|---|---|
| Sonnenschutzcreme | |
| Glycerylstearat | 3 |
| PEG-40-Stearat | 1 |
| Cetearylalkohol | 2 |
| Myristylmyristat | 1 |
| C₁₂₋₁₅ Alkylbenzoat | 5 |
| Cocosglyceride | 2 |
| Cosmacol Eli (Alkyllactate) | 5 |
| Bienenwachs | 1 |
| Ethylhexylmethoxycinnamat | 7 |
| Phenylbenzimidazol sulfonsäure | 2 |
| Butylmethoxydibenzoylmethan | 2 |
| Natriumascorbylphosphat | 0,1 |
| Tocopherylacetat | 1 |
| Silydianin | 0,01 |
| Methylpropandiol | 1 |
| Phenoxyethanol | 0,5 |
| p-Hydroxybenzoesäurealkylester (Paraben) | 0,2 |
| Diazolidinylharnstoff | 0,1 |
| C₁₀₋₃₀ Alkyl/Acrylates Crosspolymer | 0,1 |
| Carrageenan | 0,1 |
| Glycerin | 7 |
| Additive (BHT, Iminodisuccinat) | 0,4 |
| Parfüm | q.s. |
| Wasser | ad 100 |

| Beispiel 4 | Gew.-% |
|---|---|
| O/W-Creme | |
| PEG-100 Stearat | 1 |
| Glycerylstearat | 2 |
| Stearylalkohol | 2 |
| Cetylalkohol | 2 |
| C₁₂₋₁₅ Alkylbenzoat | 4 |
| Caprylsäure/Caprinsäuretriglyceride | 2 |
| Macadamiaöl | 1 |
| Myristylmyristat | 2 |
| Dimethicon | 2 |
| Isopropylpalmitat | 1 |
| Pentaerythrityl Tetraisostearate | 2 |
| Tocopherylacetat | 1 |
| Mariendistelextrakt | 0,01 |
| Kreatin | 0,1 |
| Ubichinon (Q10) | 0,03 |
| Phenoxyethanol | 0,4 |
| p-Hydroxybenzoesäurealkylester (Paraben) | 0,3 |
| lodopropinylbutylcarbamat | 0,02 |
| Cyclodextrin | 0,3 |
| Iminodisuccinat | 0,2 |
| Carragenan | 0,3 |
| Glycerin | 5 |
| Butylenglycol | 3 |
| Lecithin | 0.03 |
| Additive (SiO₂, Talkum) | 0,5 |
| Parfüm | q.s. |
| Taurin | 0.5 |
| Wasser | ad 100 |

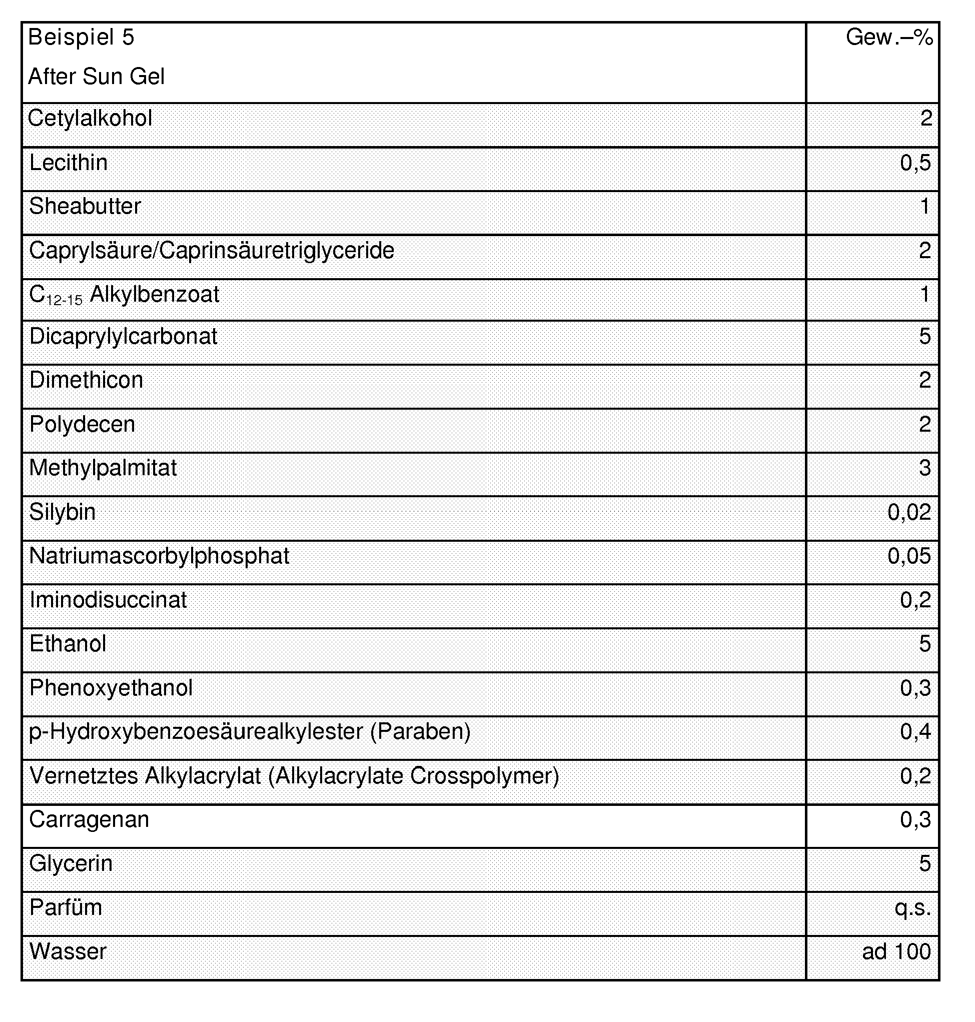

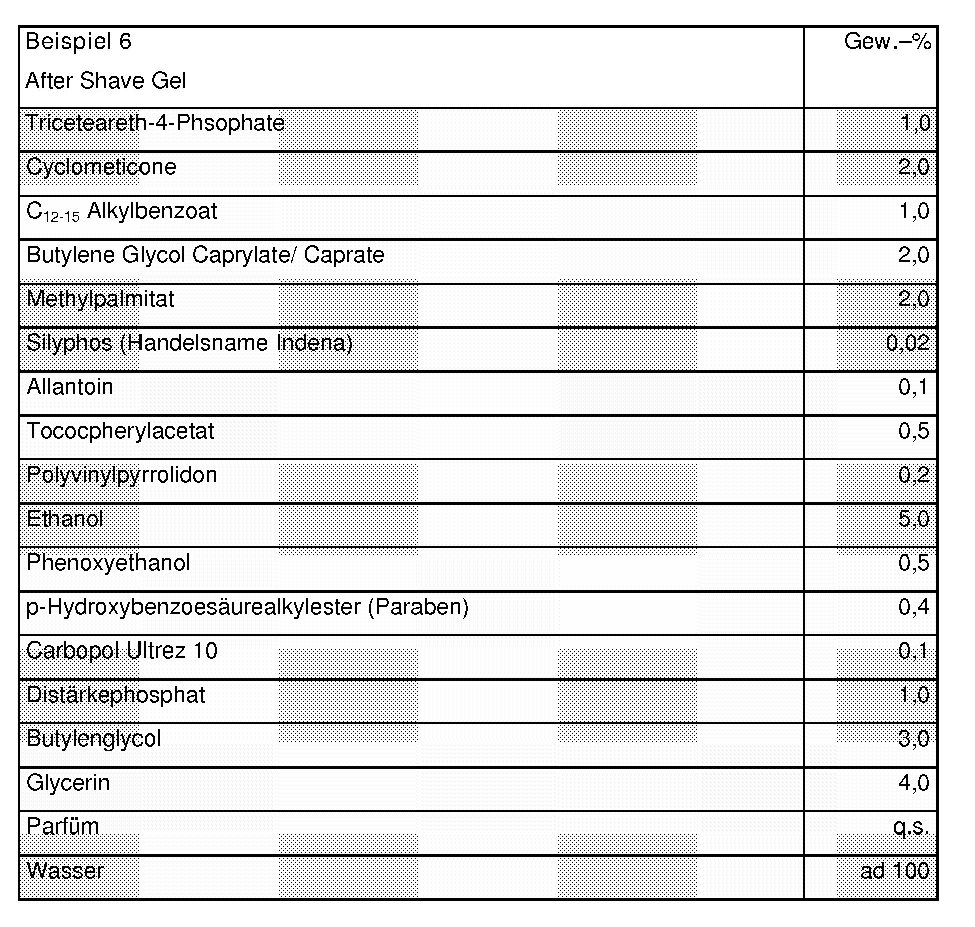

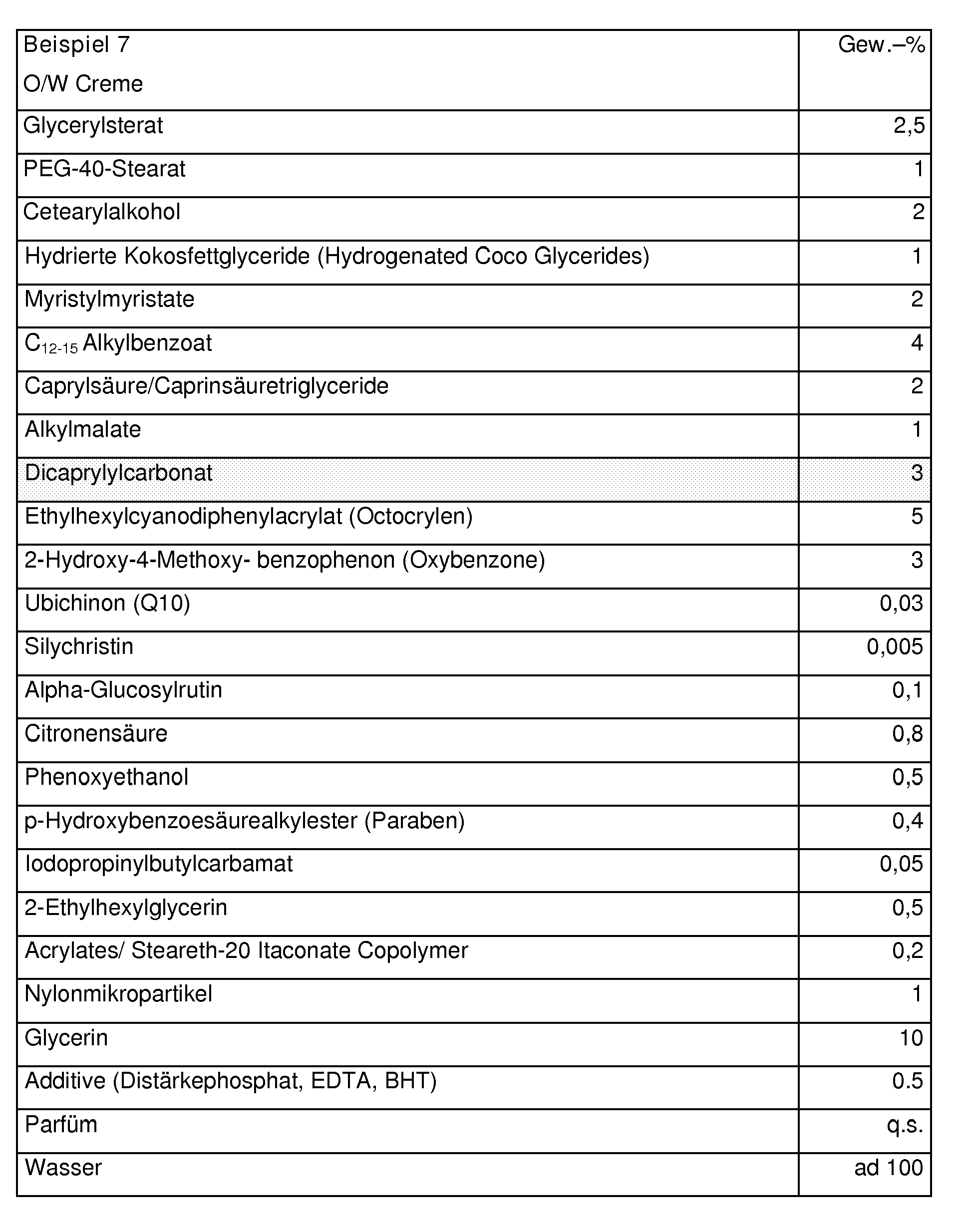

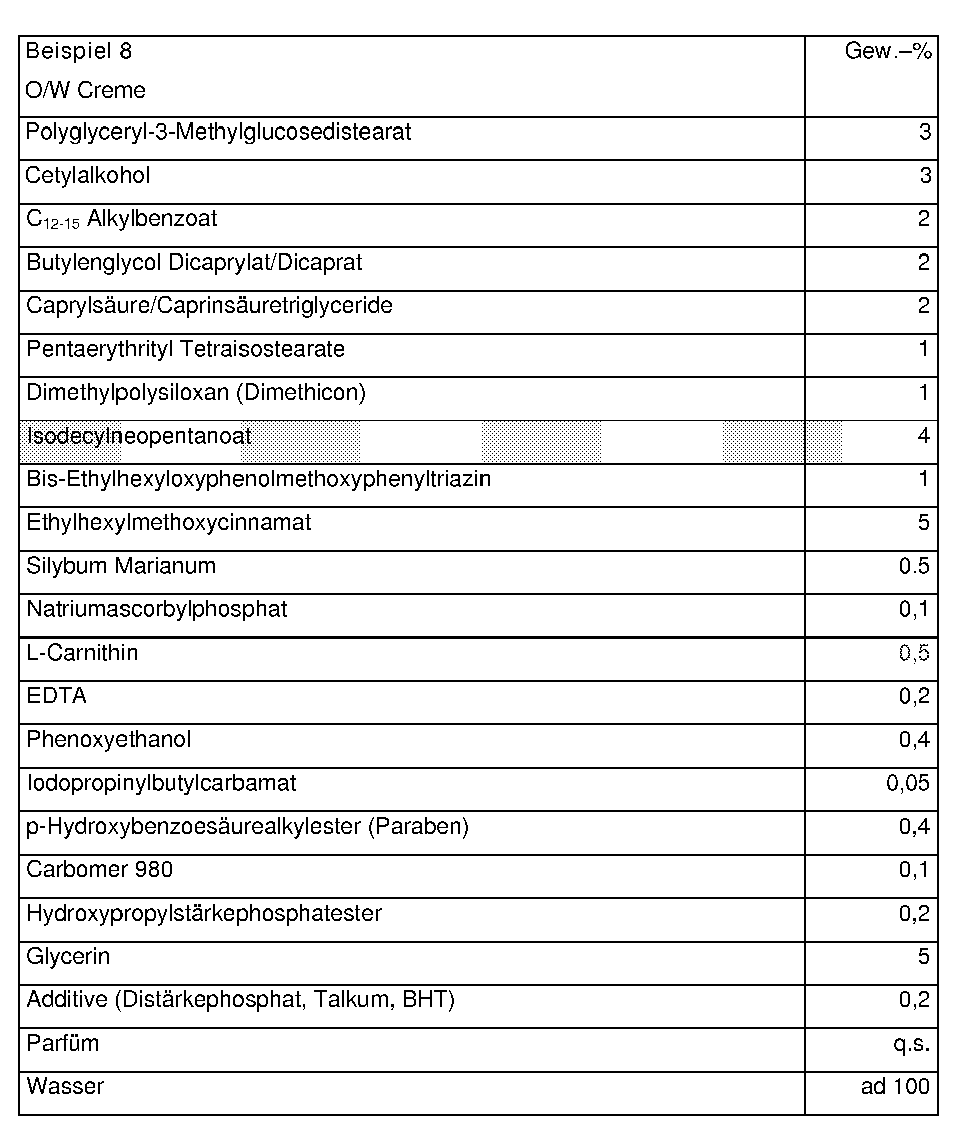

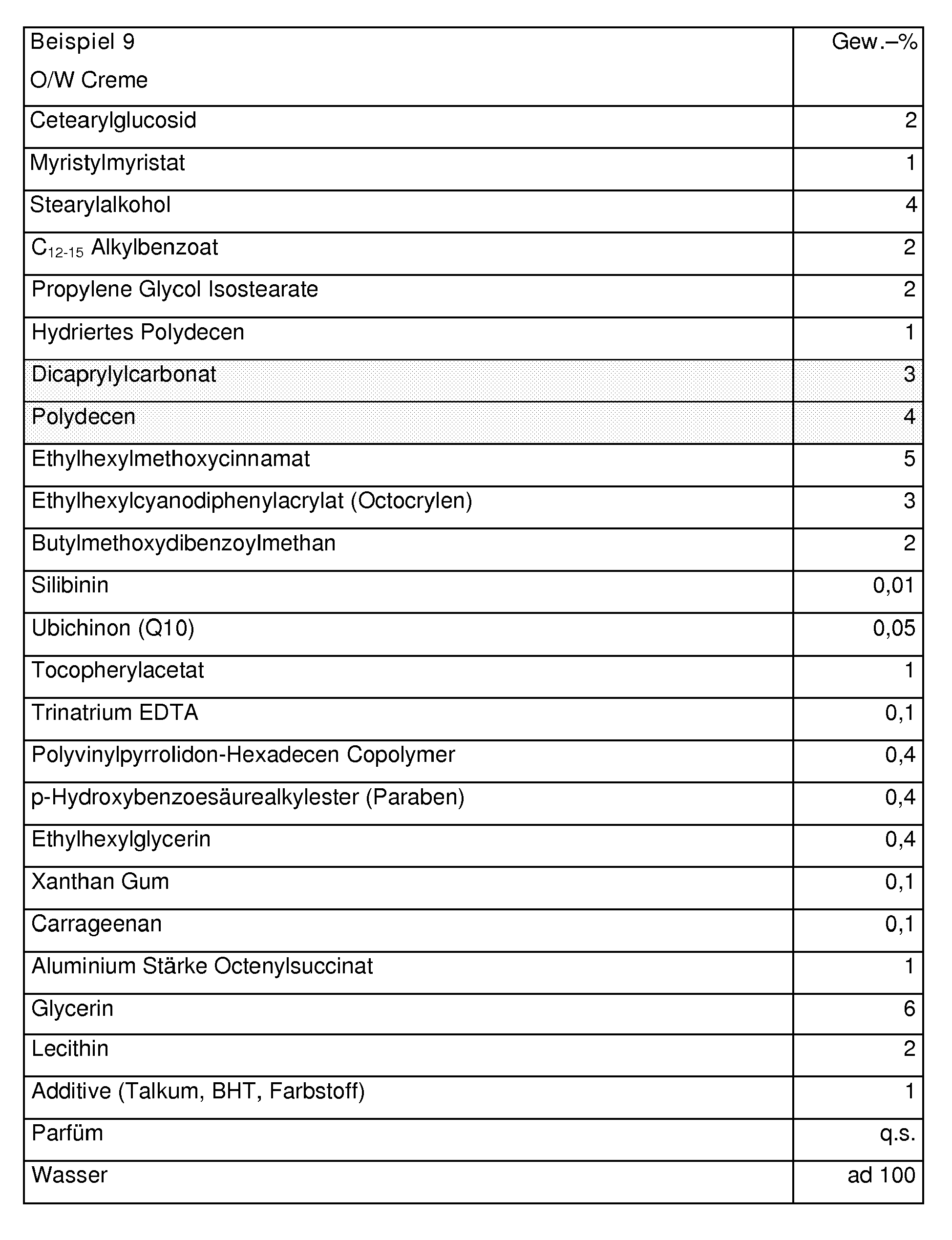

| Beispiel 10 | Gew.-% |
|---|---|
| W/O-Creme | |
| Polyglyceryl-3-Diisostearat | 5,0 |
| Polyglyceryl-2-Dipolyhydroxystearat | 2,5 |
| Cetearylalkohol | 2 |
| Cetylalkohol | 2 |
| C₁₂₋₁₅ Alkylbenzoat | 8 |
| Caprylsäure/Caprinsäuretriglyceride | 6 |
| Octyldodecanol | 5 |
| Octamethyltetrasiloxan (Cyclomethicon) | 2 |
| Niacinamid | 0,2 |
| Milchsäure | 1 |
| Citronensäure, Natriumsalz | 0,5 |
| Butylmethoxydibenzoylmethan | 1 |
| Ethylhexyltriazon | 1 |
| Ethylhexylmethoxycinnamat | 5 |
| Silyphos | 0,01 |
| Natriumpolyacrylat | 0,15 |
| Retinylpalmitat | 0,05 |
| p-Hydroxybenzoesäurealkyleste (Paraben) | 0,1 |
| Glycerin | 7 |
| Füllstoffe (EDTA, BHT) | 0,6 |
| Parfüm | q.s. |
| Wasser | Ad 100 |

| Beispiel 11 | Gew.-% |
|---|---|
| Mikroemulsion | |
| Lecithin | 2 |
| PEG-50 Hydrogenated Castor Oil Isostearat | 5 |
| Isopropylpalmitat | 2 |
| Dicaprylyl Ether | 5 |
| p-Hydroxybenzoesäurealkylester (Paraben) | 0,1 |
| Diazolidinylharnstoff | 0,2 |
| 2-Ethylhexylglycerin | 0,5 |
| Triacontyl PVP | 0,3 |
| Silybum Marianum | 0,01 |
| Cetylhydroxyethylcellulose | 0,001 |
| Glycerin | 7 |
| Butylenglycol | 3 |
| Additive (Talkum, BHT, EDTA) | 0.5 |
| Parfüm | q.s. |
| Wasser | ad 100 |

| Beispiel 12 | Gew.-% |
|---|---|
| Pickering-Emulsion | |
| Mikrokristallines Wachs | 4,5 |
| Carnaubawachs | 1,5 |
| C₁₂₋₁₅ Alkylbenzoat | 4,0 |
| C₁₂-₁₃ Alkyllakate | 4,0 |
| Bis-Diglyceryl Polyacyladipat-2 | 3,5 |
| Dimethicon | 1,0 |
| Isopropyl Palmitat | 3,5 |
| Triisostearin | 3,0 |
| Myristyl Lactat | 4,0 |
| Jojobaöl | 2,0 |
| Hydrogeniertes Polydecen | 2,5 |
| Octyldodecanol | 2,5 |
| Silybum Marianum | 0,01 |
| Lecithin | 0,2 |
| Ethylhexyl Methoxycinnamat | 2 |
| Butyl Methoxydibenzoylmethan | 0,5 |
| Mikronisiertes Titandioxid (Eusolex T 2000) | 2,0 |
| Titandioxid Cl 77891 | 4,0 |
| Eisenoxide Cl 77491, 77492, 77499 | 3,2 |
| D&C Rot 7 | 0,6 |
| Tocopheryl Acetat | 1,0 |
| Xylitol | 2,0 |
| EDTA | 0,2 |
| Glycerin | 5,0 |
| Konservierungsmittel, BHT, Parfum, Aroma | q.s. |
| Wasser | 30,0 |
| Ricinusöl | ad 100 |

| Beispiel 13 | Gew.-% |
|---|---|
| W/O- Pflegestift | |
| Caprylsäure/Caprinsäuretriglyceride | 8 |
| Octyldodecanol | 7 |
| Paraffinöl | 2 |
| Pentaerythrityltetraisostearat | 2 |
| C₁₂₋₁₅ Alkylbenzoat | 2 |
| Jojobaöl | 2 |
| PEG-45/ Dodecyl Glycol Copolymer | 3 |
| Polyglyceryl-3-diisostearat | 2,5 |
| Saccharosedistearat | 0,5 |
| Bis-Diglycerylpolyacyladipate-2 | 9 |
| Cetyl Palmitate | 2,5 |
| C₁₆₋₃₆-Alkylstearat | 14 |
| Carnaubawachs | 1,5 |
| Bienenwachs | 0,5 |
| Ethylhexylcyanodiphenylacrylat (Octocrylen) | 2 |
| Silyphos | 0,11 |
| Phenoxyethanol | 0,2 |
| Carbopol 981 | 0,1 |
| Wismutoxychlorid (BiOCl) | 2 |
| PTFE | 2,5 |
| Perlglanzpigmente | 3 |
| Rokonsal S1 | 0,4 |
| Glycerin | 5 |
| Parfüm, BHT, Neutralisationsmittel, | q.s |
| Wasser | ad 100 |

| Beispiel 14 | Gew.-% |
|---|---|
| Füllmasse für Gelatinekapseln | |
| Flavonolignane aus der Mariendistel | 75 |
| Pflanzenöl | 10 |
| Neutralöl | 10 |
| Lecithin | 5 |

| Beispiel 15 | Gew.-% |
|---|---|
| W/O- Abdeckstift | |
| Caprylsäure/Caprinsäuretriglyceride | 5 |
| Octyldodecanol | 5 |
| Pentaerythrityltetraisostearat | 4 |
| Dimethicone | 0,5 |
| PEG-45/ Dodecyl Glycol Copolymer | 3,5 |
| Bis-Diglycerylpolyacyladipate-2 | 2 |
| C₁₆₋₃₆-Alkylstearat | 1 |
| C₂₀₋₄₀-Alkylstearat | 8 |
| Carnaubawachs | 1,5 |
| PVP / Eicosene Copolymer | 1 |
| Mikronisiertes Titandioxid | 2 |
| Octylmethoxycinnamat | 2 |
| Silyphos | 0,12 |
| Phenoxyethanol | 0,4 |
| Carbopol 981 | 0,15 |
| Nylon-12 | 3 |
| Lauroyl Lysine | 0,5 |
| PMMA | 6 |
| Titandioxid mit Al₂O₃ beschichtet | 7 |
| Eisenoxide | 4 |
| Ultramarin | 0,5 |
| Germall II | 0,25 |
| Glycerin | 2 |
| Parfüm, BHT, Neutralisationsmittel, | q.s |
| Wasser | ad 100 |

| Beispiel 16 | Gew.-% |
|---|---|
| W/O- Foundationstift | |
| Caprylsäure/Caprinsäuretriglyceride | 5 |
| Octyldodecanol | 5 |
| Dicaprylylcarbonat | 3 |
| Dicaprylylether | 2 |
| Dimethicone | 0,5 |
| PEG-45/ Dodecyl Glycol Copolymer | 2 |
| Polyglyceryl-3-diisostearat | 1,5 |
| C₁₆₋₃₆-Alkylstearat | 2 |
| C₂₀₋₄₀-Alkylstearat | 8 |
| Silyphos | 0,2 |
| Phenoxyethanol | 0,3 |
| Carbopol 981 | 0,2 |
| Wismutoxychlorid (BiOCl) | 3 |
| Polymethylsilsesquioxane (Tospearl) | 0,5 |
| PMMA | 3 |
| Titandioxid mit Al₂O₃ beschichtet | 6 |
| Eisenoxide | 4 |
| Ultramarin | 0,6 |
| Glycerin | 10 |
| Parfüm, BHT, Neutralisationsmittel, | q.s |
| Wasser | ad 100 |

| Beispiel 17 | Gew.-% |
|---|---|
| W/O- Sonnenschutzstift | |
| Caprylsäure/Caprinsäuretriglyceride | 8 |
| Octyldodecanol | 8 |
| Pentaerythrityltetraisostearat | 8 |
| Jojobaöl | 1 |
| Polyglyceryl-3-diisostearat | 2 |
| PEG-30 Di-Polyhydroxystearat | 2,5 |
| C₁₆₋₃₆-Alkylstearat | 1 |
| C₂₀₋₄₀-Alkylstearat | 9 |
| PVP / Eicosene Copolymer | 1 |
| Butyl Methoxydibenzoylmethane | 1 |
| Mikronisiertes Titan Dioxid | 4 |
| Ethylhexylcyanodiphenylacrylat (Octocrylen) | 3,6 |
| Octylmethoxycinnamat | 3,6 |
| Extrakt aus Silybum Marianum | 0,001 |
| Phenoxyethanol | 0,4 |
| Lecithine | 0,17 |
| Bornitrid | 3 |
| Polymethylsilsesquioxane (Tospearl) | 1 |
| Silica LDP | 1 |
| Glydant Plus | 0,3 |
| Glycerin | 5 |
| Parfüm, BHT, Neutralisationsmittel, | q.s |
| Wasser | ad 100 |

## Patentansprüche

1. Wasserhaltige kosmetische Zubereitung enthaltend mindestens einen schwerlöslichen Wirkstoff aus der Gruppe der Flavonolignane, mindestens ein bei Raumtemperatur flüssiges, synthetisches Ester-Öl mit besonders hohem Gehalt an Esterbindungen und einen hydrophilen Emulgator aus der Gruppe Zuckerderivate, PEG-Ester und / oder Lecithine.

2. Zubereitung nach einem der vorangehenden Patentansprüche **dadurch gekennzeichnet, dass** der oder die schwerlöslichen Wirkstoffe aus der Gruppe der Flavonolignane Silymarin, Silibinin, Silychristin, Isosilybin, Silydianin, besonders bevorzugt Silymarin und/oder Silibinin sind.

3. Zubereitung nach einem der vorangehenden Patentansprüche **dadurch gekennzeichnet, dass** der schwerlösliche Wirkstoff aus der Gruppe der Flavonolignane in Konzentrationen von 0,0001 bis 5 Gew.-% bezogen auf das Gewicht der gesamten Zubereitung vorliegen.

4. Zubereitung nach einem der vorangehenden Patentansprüche **dadurch gekennzeichnet, dass** das oder die bei Raumtemperatur flüssigen, synthetischen Ester-Öle mit besonders hohem Gehalt an Esterbindungen aus der Gruppe der Alkylbenzoate, Alkyllaktate, Alkylmalate, Isopropylpalmitat, Propylene Glycol Isostearate, Propylene Glycol Monolaurate, Butylene Glycol Caprylate/ Caprate, Dibutyl Adipate, Propylene Glycol Monolaurate, Pentaerythrityl Tetraisostearate gewählt werden.

5. Zubereitung nach einem der vorangehenden Patentansprüche **dadurch gekennzeichnet, dass** das oder die bei Raumtemperatur flüssigen, synthetischen Ester-Öle mit besonders hohem Gehalt an Esterbindungen in Konzentrationen von 1 bis 30 Gew.-% bezogen auf das Gewicht der gesamten Zubereitung vorliegen.

6. Zubereitung nach einem der vorangehenden Patentansprüche **dadurch gekennzeichnet, dass** der hydrophile Emulgator aus der Gruppe Cetearylglucoside, Polyglycerol-3 Methyl Glucose Distearate, PEG- Ester wie PEG-40-Stearate und/oder Lecithine gewählt wird.

7. Zubereitung nach einem der vorangehenden Patentansprüche **dadurch gekennzeichnet, dass** die Zubereitung mindestens 5 Gew.-% Öl enthält, bezogen auf das Gewicht der gesamten Zubereitung.

8. Zubereitung nach einem der vorangehenden Patentansprüche **dadurch gekennzeichnet, dass** die Zubereitung eine Emulsion ist.

9. Zubereitung nach einem der vorangehenden Patentansprüche **dadurch gekennzeichnet, dass** ein sprühgetrockneter Extrakt eingesetzt wird.

10. Zubereitung nach einem der vorangehenden Patentansprüche zur oralen Applikation.

11. Verwendung von bei Raumtemperatur flüssigen synthetischen Ester-Ölen mit besonders hohem Gehalt an Esterbindungen und hydrophilen Emulgatoren aus der Gruppe Zuckerderivate oder Lecithine zur Verbesserung der Löslichkeit und/oder Verringerung der Kristallisationsneigung von in wasserhaltigen Zubereitungen schwerlöslichen Wirkstoffe aus der Gruppe der Flavonolignanen, bevorzugt Silymarin, Silibinin, Silychristin, Isosilybin, Silydianin, besonders bevorzugt Silymarin und/oder Silibinin in wasserhaltigen Zubereitungen.
